# EUROPÄISCHE PATENTSCHRIFT

(11) **EP 3 841 422 B1**
(45) Veröffentlichungstag und Bekanntmachung des Hinweises auf die Patenterteilung: **01.04.2026**
(21) Anmeldenummer: 19762910.8
(22) Anmeldetag: 20.08.2019
(51) Int. Cl.: G02B 21/32, C12M 1/42, C12M 1/34, G01N 15/14, G01N 15/10

(54) **VORRICHTUNG UND VERFAHREN ZUR DETEKTION EINER RÄUMLICHEN STRECKUNG VON MINDESTENS EINER ADHÄRENTEN BIOLOGISCHEN ZELLE**
APPARATUS AND METHOD FOR DETECTING A SPATIAL ELONGATION OF AT LEAST ONE ADHERENT BIOLOGICAL CELL
DISPOSITIF ET PROCÉDÉ POUR DÉTECTER UNE EXTENSION SPATIALE D'AU MOINS UNE CELLULE BIOLOGIQUE ADHÉRENTE

(30) Priorität: 20.08.2018 DE 102018213965
(43) Veröffentlichungstag der Anmeldung: 30.06.2021
(73) Patentinhaber: Universität Ulm, 89081 Ulm (DE)
(72) Erfinder: NECKERNUSS, Tobias, 73054 Eislingen (DE); GEIGER, Daniel, 88441 Mittelbiberach (Reute) (DE); MARTI, Othmar, 89075 Ulm (DE)
(74) Vertreter: Pfenning, Meinig & Partner mbB
(86) Internationale Anmeldenummer: PCT/EP2019/072212
(87) Internationale Veröffentlichungsnummer: WO 2020/038914

(56) Entgegenhaltungen:
- EP-A2- 1 469 483
- DE-A1- 19 954 933
- US-A- 6 067 859
- US-A1- 2013 230 879
- US-A1- 2018 188 173
- SEN S ET AL: "Combining mechanical and optical approaches to dissect cellular mechanobiology", JOURNAL OF BIOMECHANICS, PERGAMON PRESS, NEW YORK, NY, US, vol. 43, no. 1, 5 January 2010 (2010-01-05), pages 45 - 54, XP026814519, ISSN: 0021-9290, [retrieved on 20091012]
- SERGEY ERMILOV ET AL: "Measurements of cell wall mechanical properties using optically trapped fluorescent microspheres", PROCEEDINGS OF SPIE, vol. 5514, 18 October 2004 (2004-10-18), 1000 20th St. Bellingham WA 98225-6705 USA, pages 189, XP055647271, ISSN: 0277-786X, ISBN: 978-1-5106-2687-4, DOI: 10.1117/12.559717

## Beschreibung

Es wird eine Vorrichtung zur Detektion einer räumlichen Streckung von mindestens einer adhärenten biologischen Zelle bereitgestellt. Die Vorrichtung enthält mindestens eine biologische Zelle, die auf einem Substrat adhäriert ist, einen Laser zur Bestrahlung der mindestens einen biologischen Zelle zur räumlichen Streckung der Zelle in eine Richtung parallel zur Bestrahlungsrichtung und einen Detektor zur Detektion der räumlichen Streckung der Zelle in der Richtung parallel zur Bestrahlungsrichtung. Ferner wird ein entsprechendes Verfahren zur räumlichen Streckung einer adhärenten biologischen Zelle bereitgestellt und die Verwendungen der Vorrichtung und des Verfahrens vorgeschlagen. Es ist mit der Vorrichtung und dem Verfahren möglich, von Teilen von adhärenten Zellen bis hin zu ganzen Gruppen von adhärenten Zellen räumlich selektiv, zeitlich selektiv und kontaktlos die mechanischen Eigenschaften in dem natürlichen, adhärenten Zustand der Zelle(n) zu ermitteln.

Im Stand der Technik sind zahlreiche Verfahren zur Untersuchung der mechanischen Eigenschaften von adhärenten biologischen Zellen bekannt. Beispiele hierfür sind die Raster-Kraft-Mikroskopie (engl. "atomic force microscopy), das Mikropipettieren (engl. "micropipetting"), die optische Falle (engl. "optical trap") und die mechanische Verstreckung des Substrats.

Nachteil an der Raster-Kraft-Mikroskopie, dem Mikropipettieren und der mechanischen Verstreckung des Substrats ist, dass die Verstreckungskraft auf die biologischen Zellen über Etablierung eines Kontakts zu den biologischen Zellen ausgeübt wird, also auf eine in gewissem Grad "invasive" Art und Weise. Nachteilig ist hierbei, dass die Kontaktierung lokal an einer bestimmten Oberfläche einer Zelle eine hohe Kraft ausübt, wodurch die Zelle während der Messung beschädigt werden kann und die Messung verfälscht werden kann.

Mit der kontaktlosen Messung der mechanischen Eigenschaften von biologischen Zellen über den im Stand der Technik bekannten optischen Strecker (engl. "optical stretcher") ist der Nachteil verbunden, dass die Zellen für die Erzeugung der Streckung in einem flüssigen Medium (z.B. einem wässrigen Puffer) suspendiert sein müssen.

Beispielsweise ist aus der US 6,067,859 A ein Messverfahren bekannt, bei der in einen wässrigen Medium suspendierten biologische Zellen über Einwirken von elektromagnetischer Strahlung eine sog. optische Streckung (engl. "optical stretching") erfahren und daraus mechanische Eigenschaften der Zellen abgeleitet werden. Für die Messung ist es nötig, die zu untersuchende biologische Zelle über elektromagnetische Strahlung aus zwei gegenüberliegenden Strahlungsquellen (z.B. Laser) über die Wirkung der elektromagnetischen Strahlung zu immobilisieren, d.h. an dem Ort "gefangen" zu halten, an dem die entgegengesetzt gerichtete elektromagnetische Strahlung auf die jeweils gegenüberliegenden Seiten der biologischen Zelle trifft. Da der Brechungsindex des wässrigen Mediums, in dem die Zellen suspendiert sind, kleiner ist als der Brechungsindex der Zellen, bewirkt die auf die suspendierte(n) Zelle(n) einwirkende elektromagnetische Strahlung eine Streckung der suspendierte(n) Zelle(n) in zwei entgegengesetzte Richtungen entlang einer Gerade, die mit der optischen Achse der beiden gegenüberliegenden Strahlungsquellen zusammenfällt. Über die Geschwindigkeit und das Ausmaß der Streckung können mechanische Eigenschaften der biologischen Zellen abgeleitet werden.

Nachteil des optischen Streckers für suspendierte Zellen ist, dass mit ihm keine räumliche Streckung von adhärenten biologischen Zellen erfassbar ist. Im Falle einer Messung von Zellen, die sich durch adhärentes Wachstum auszeichnen (z.B. Fibroblasten) ist es für eine Detektion der räumlichen Streckung dieser Zellen über dieses Verfahren nötig, die adhärenten Zellen vor der Messung von dem Substrat, auf dem sie kultiviert werden, abzulösen (z.B. über Einwirkung des Enzyms Trypsin) und in einer Flüssigkeit (z.B. ein Puffer) zu resuspendieren. Da die Ablösung dieser Zellen von dem Substrat die mechanischen Eigenschaften dieser Zellen verglichen zu ihrem natürlichen, adhärenten Status verändert, ist es mit diesem Verfahren nur möglich, die mechanischen Eigenschaften dieser Zellen in einem suspendierten Zustand zu erfassen, also einem Zustand, der von ihrem natürlichen, adhärenten Zustand in *vivo* abweicht.

Ein weiterer Nachteil der Messung mit dem optischen Strecker bei suspendierten Zellen ist, dass die Strahlbreite der elektromagnetischen Strahlung breiter sein muss als die Breite der zu untersuchenden Zelle, da die zu untersuchende Zelle sonst nicht stabil zwischen den beiden Strahlen der entgegengesetzten elektromagnetischen Strahlung gefangen bleibt. Folglich ist es mit diesem Verfahren nicht möglich, eine Streckungskraft nur an einzelnen Punkten an der Oberfläche einer zu untersuchenden Zelle zu applizieren, um daraus ortsselektiv mechanische Eigenschaften an diesen Punkten abzuleiten.

Eine weitere Vorrichtung zur Manipulation von Zellen ist aus der Offenbarung der EP 1 469 483 A2 bekannt.

Ausgehend hiervon war es die Aufgabe der vorliegenden Erfindung, eine Vorrichtung und ein Verfahren zur Detektion einer räumlichen Streckung von mindestens einer adhärenten biologischen Zelle bereitzustellen, welche die Nachteile der im Stand der Technik bekannten Vorrichtungen und Verfahren nicht aufweist. Speziell sollte es mit der Vorrichtung bzw. mit dem Verfahren möglich sein, räumlich und zeitlich selektiv eine definierte Streckungskraft auf einen Teil von adhärenten biologischen Zellen, eine ganze adhärente biologische Zelle oder mehrere adhärente biologische Zellen gleichzeitig auszuüben und dabei nicht-invasiv (d.h. kontaktlos) den Grad der Streckung der biologischen Zelle(n) exakt zu erfassen, um daraus mechanische Eigenschaften der biologischen Zelle(n) abzuleiten.

Die Aufgabe wird gelöst durch die Vorrichtung mit den Merkmalen von Anspruch 1, das Verfahren mit den Merkmalen von Anspruch 8 und die Verwendung mit den Merkmalen von Anspruch 15.

Erfindungsgemäß wird eine Vorrichtung zur Detektion einer räumlichen Streckung von mindestens einer adhärenten Zelle bereitgestellt, enthaltend
a) mindestens eine biologische Zelle auf einem Substrat;
b) ein Laser zur Bestrahlung der mindestens einen biologischen Zelle mit elektromagnetischer Strahlung in einer Bestrahlungsrichtung, wobei der Laser dazu konfiguriert ist, elektromagnetische Strahlung auf die mindestens eine biologische Zelle zu strahlen, die eine räumliche Streckung der mindestens einen biologischen Zelle in eine Richtung parallel zur Bestrahlungsrichtung bewirkt; und
c) ein Detektor zur Detektion einer räumlichen Streckung der mindestens einen biologischen Zelle in einer Richtung parallel zur Bestrahlungsrichtung;
wobei die mindestens eine biologische Zelle eine auf dem Substrat adhärente biologische Zelle ist, dadurch gekennzeichnet, dass der Laser dazu konfiguriert ist, die elektromagnetische Strahlung mit einer Leistung im Bereich von 50 bis 2000 mW abzustrahlen, wobei die Vorrichtung keinen Laser aufweist, der dazu konfiguriert ist, elektromagnetische Strahlung auf die mindestens eine biologische Zelle in einer weiteren Richtung zu strahlen, die einen Winkel von 180° zur Bestrahlungsrichtung aufweist.

Die auf dem Substrat adhärenten Zellen weisen einen Brechungsindex auf (z.B. einen Brechungsindex von 1,38), der sich von ihrer Umgebung, d.h. dem darunterliegenden Medium (z.B. Glassubstrat mit einem Brechungsindex von 1,52) und dem darüberliegenden Medium (z.B. Luft mit einem Brechungsindex von 1,00 oder wässriger Puffer mit einem Brechungsindex von 1,33), unterscheidet. Die Einwirkung von elektromagnetischer Strahlung über den Laser bewirkt, dass auf die adhärenten Zellen entlang der optischen Achse der Strahlung eine Streckungskraft wirkt, die in Richtung des umgebenden Mediums mit einem niedrigeren Brechungsindex (d.h. in Richtung Luft oder dem wässrigen Medium) gerichtet ist. Folglich werden die Zellen durch die Laserstrahlung vom Substrat weg gestreckt, wobei die Adhäsionskraft der Zellen auf dem Substrat verhindert, dass die von der Laserstrahlung bewirkte Streckung der Zellen zu einem Ablösen der Zellen von dem Substrat führt. Die erzeugte räumliche Streckung bzw. relative Höhenänderung der auf dem Substrat adhärent bleibenden Zelle kann über den Detektor erfasst werden.

Es ist über die erfindungsgemäße Vorrichtung erstmalig möglich, einen Teil von adhärenten biologischen Zellen, eine ganze adhärente biologische Zelle oder mehrere adhärente biologische Zellen räumlich selektiv, zeitlich selektiv und ohne Kontaktierung mit einem Messgerät über eine gezielt applizierte Streckungskraft in eine Richtung senkrecht zum Substrat und vom Substrat weg zu Verformen, den Grad der Verformung zu detektieren und aus dem Grad der Verformung mechanische Eigenschaften (z.B. viskoelastischen Eigenschaften über Spannungs-Dehnungskurven) der mindestens einer adhärenten Zelle, oder Teilen davon, in dem natürlichen (d.h. adhärenten) Zustand der Zelle abzuleiten.

Weist die vom Substrat abgewandte Umgebung der mindestens einen biologischen Zelle einen Brechungsindex auf, der kleiner ist als der Brechungsindex der Zelle selbst (z.B. Luft mit Brechungsindex 1,00 oder wässriger Puffer mit Brechungsindex 1,33), wird die mindestens eine Zelle vom Substrat weg gestreckt. Weist die vom Substrat abgewandte Umgebung der mindestens einen biologischen Zelle einen Brechungsindex auf, der größer ist als der Brechungsindex der Zelle selbst (z.B. Glycerin mit Brechungsindex 1,47), wird die mindestens eine Zelle zum Substrat hin gestreckt, d.h. zum Substrat hin gestaucht. In beiden Fällen lassen sich durch den detektierten Grad der Verformung der mindestens einen adhärenten Zelle mechanische Eigenschaften der mindestens einen adhärenten Zelle, oder Teilen davon, ableiten.

Die erfindungsgemäße Vorrichtung kann dadurch gekennzeichnet sein, dass die Vorrichtung keinen Laser aufweist, der dazu konfiguriert ist, elektromagnetische Strahlung auf die mindestens eine biologische Zelle in einer weiteren Richtung zu strahlen, die einen Winkel im Bereich von > 90° bis 180° zur Bestrahlungsrichtung aufweist.

In einer bevorzugten Ausgestaltungsform enthält die Vorrichtung eine Lichtquelle zur Bestrahlung der mindestens einen biologischen Zelle mit Licht in einer Lichtbestrahlungsrichtung, wobei die Lichtbestrahlungsrichtung bevorzugt parallel zur Bestrahlungsrichtung des Lasers auf die mindestens eine biologische Zelle ist und/oder der Bestrahlungsrichtung des Lasers auf die mindestens eine biologische Zelle entgegen gerichtet ist (d.h. in einem Winkel von 180° zur Bestrahlungsrichtung des Lasers auf die mindestens eine biologische Zelle strahlt). Die Lichtquelle ist bevorzugt ausgewählt aus der Gruppe bestehend aus Lichtquellen von inkohärentem Licht, besonders bevorzugt ausgewählt aus der Gruppe bestehend aus Halogenlampe, LED und Kombinationen hiervon.

Die Vorrichtung ist bevorzugt so konfiguriert, dass die elektromagnetische Strahlung, optional abgelenkt über einen dichroitischen und/oder einen einfachen Spiegel, senkrecht zu einer Oberfläche des Substrats, auf der sich die mindestens eine biologische Zelle befindet, auf die mindestens eine biologische Zelle (bzw. Teile davon) strahlt. Der dichroitische Spiegel kann dazu geeignet sein, Licht mit einer Wellenlänge > 700 nm (z.B. Laserlicht der Wellenlänge 800 nm) zu reflektieren und Licht mit einer Wellenlänge von ≤ 700 nm (z.B. sichtbares Licht) passieren zu lassen. Der einfache Spiegel kann dazu geeignet sein, sichtbares Licht und IR-Licht (z.B. Licht mit einer Wellenlänge von 400 nm bis 800 nm) zu reflektieren.

In einer besonders bevorzugten Ausführungsform ist die Vorrichtung so konfiguriert, dass die elektromagnetische Strahlung durch das Substrat auf die mindestens eine biologische Zelle (oder Teile davon) strahlt. In diesem Fall ist es essentiell, dass das Substrat die elektromagnetische Strahlung des Lasers zumindest teilweise passieren lässt, wobei das Substrat besonders bevorzugt transparent für die elektromagnetische Strahlung ist. Das Substrat kann ausgewählt sein aus der Gruppe bestehend aus Glas, Quarzglas, Quarz, transparenter Kunststoff (z.B. PMMA oder Polycarbonat) und Kombinationen hiervon. Ferner kann die Vorrichtung dazu konfiguriert sein, den Laser entlang einer Oberfläche des Substrats zu bewegen und/oder das Substrat entlang einer Oberfläche des Substrats zu bewegen, bevorzugt entlang einer Ebene, die parallel zu einer Oberfläche des Substrats ist. Die Bewegung des Substrats kann über einen Piezo-Tisch erfolgen, auf dem sich das Substrat befindet.

Die Vorrichtung kann eine Recheneinheit aufweisen, die bevorzugt dazu konfiguriert ist, Daten des Detektors und/oder eines Datenspeichers der Vorrichtung zu empfangen und auszuwerten, wobei die Recheneinheit bevorzugt dazu konfiguriert ist, eine Umwandlung der Daten zu einer räumlichen Streckung der mindestens einen Zelle in unbekannter Länge in eine räumliche Streckung der mindestens einen Zelle in bekannter Länge umzuwandeln, wobei die Recheneinheit besonders bevorzugt dazu konfiguriert ist, für die Umwandlung am Detektor detektierte Beugungsbilder und eine (kalibrierte) Punktspreizfunktion zu nutzen und die Recheneinheit ganz besonders bevorzugt dazu konfiguriert ist, die Daten zur räumlichen Streckung in eine fokale Höhe, ein E-Modul und/oder Schubmodul der mindestens einen biologischen Zelle umzuwandeln.

Für eine Erfassung der relativen Höhenänderung der mindestens einen Zelle mit hoher Sensitivität ist es vorteilhaft, wenn die Zellmembran der mindestens einen Zelle mit einer Substanz markiert ist, die einen größeren Brechungsindex aufweist als die von dem Substrat abgewandten Umgebung der mindestens einen Zelle (z.B. > 1,33 im Fall eines wässrigen Puffers), bevorzugt einen Brechungsindex, der größer ist als der Brechungsindex der mindestens einen Zelle (z.B. > 1,38). Beispielsweise können hierfür an die Zellmembran der Zelle(n) Partikel adhäriert sein. Die Partikel können beispielsweise Kunststoff, bevorzugt Polystyrol (Brechungsindex ca. 1,58), enthalten oder daraus bestehen. Bevorzugt haben die Partikel einen mittleren Partikeldurchmesser im Bereich von 1 nm bis 10 µm, besonders bevorzugt 10 nm bis 5 µm, ganz besonders bevorzugt 100 nm bis 2 µm, insbesondere 1 µm, gemessen über dynamische Lichtstreuung. Insbesondere ist die mindestens eine Zelle mit 1 bis 10, bevorzugt 2 bis 9, besonders bevorzugt 4 bis 8, Partikeln markiert. Die Detektionssensitivität der Streckung der mindestens einen Zelle lässt sich somit erheblich steigern, da die Bewegung der Partikel über den Detektor erfasst werden kann und nicht die Bewegung der (unmarkierten) Zellmembran über den Detektor erfasst werden muss.

Zur Umwandlung der vom Detektor erfassten relativen Höhenänderung in eine absolute Höhenänderung ist der Detektor in einer bevorzugten Ausgestaltungsform der Vorrichtung kalibrierbar. In dieser Hinsicht ist das Substrat der Vorrichtung bevorzugt in einer Richtung parallel zur Bestrahlungsrichtung des Lasers bewegbar, besonders bevorzugt über einen Piezo-Tisch, den die erfindungsgemäße Vorrichtung enthält und auf dem das Substrat angeordnet ist. Durch die Beweglichkeit des Substrats in eine Richtung parallel zur Bestrahlungsrichtung des Lasers kann jedem Detektorsignal ein bestimmter Wert der Höhenänderung zugeordnet werden und somit die an sich nur qualitative Höhenänderung der Zelle(n) quantifiziert werden (z.B. in Nanometer bis Mikrometer angegeben werden).

In einer weiteren bevorzugten Ausgestaltungsform enthält die Vorrichtung eine Temperiereinheit zur Temperierung des Substrats. Bevorzugt ist die Temperiereinheit dazu konfiguriert, das Substrat auf eine Temperatur im Bereich von >0°C bis <60°C, bevorzugt 5°C bis 50 °C, besonders bevorzugt 10°C bis 45°C, insbesondere 20°C bis 40°C, zu temperieren. Das Substrat kann ein Kanalsystem enthalten, das von einer Kühlflüssigkeit der Temperiereinheit durchströmbar ist. Ferner kann das Substrat eine spezifische Wärmekapazität von weniger als 4 J/g·K, bevorzugt weniger als 3 J/g·K, besonders bevorzugt weniger als 2 J/g·K, ganz besonders bevorzugt weniger als 1 J/g·K, insbesondere weniger als 0,5 J/g·K, aufweisen. Je niedriger die spezifische Wärmekapazität des Substrats ist, desto schneller lässt sich die Temperatur des Substrats und damit der auf dem Substrat adhärenten Zellen ändern. Es kann somit das mechanische Verhalten biologischer Zellen bei schnellen Temperatursprüngen beobachtet werden.

Die Vorrichtung, insbesondere der Laser der Vorrichtung, kann dazu konfiguriert sein, die elektromagnetische Strahlung mit einer Wellenlänge im Bereich von 400 bis 1200 nm, bevorzugt im Bereich von 500 bis 1100 nm, besonders bevorzugt im Bereich von 600 bis 1000 nm, insbesondere im Bereich von 700 bis 900 nm, abzustrahlen.

Ferner kann der Laser der Vorrichtung dazu konfiguriert sein, die elektromagnetische Strahlung mit einer Leistung im Bereich von 200 bis 2000 mW, ganz besonders bevorzugt 1000 bis 2000 mW, abzustrahlen.

Zudem kann die Vorrichtung, insbesondere der Laser der Vorrichtung, dazu konfiguriert sein, die elektromagnetische Strahlung gepulst, bevorzugt mit einer Frequenz von 1 bis 1000 Hz, abzustrahlen.

In einer bevorzugten Ausgestaltungsform ist die Vorrichtung, insbesondere der Laser der Vorrichtung, dazu konfiguriert, die elektromagnetische Strahlung für einen Zeitraum im Bereich von 0,05 bis 15 Sek., bevorzugt 0,2 bis 10 Sek., besonders bevorzugt 0,5 bis 5 Sek., insbesondere 1 bis 2 Sek., abzustrahlen.

Die Vorrichtung, insbesondere der Laser der Vorrichtung, kann dazu konfiguriert sein, die elektromagnetische Strahlung in einem Winkel im Bereich von >0° bis 90°, bevorzugt im Bereich von 30° bis 90°, besonders bevorzugt im Bereich von 60° bis 90°, ganz besonders bevorzugt im Bereich von 80° bis 90°, insbesondere 90°, zu einer Oberfläche des Substrats abzustrahlen.

Ferner kann die Vorrichtung, insbesondere der Laser der Vorrichtung, dazu konfiguriert sein, die elektromagnetische Strahlung mit einer Strahlenbreite im Bereich von 0,1 bis 800 µm, bevorzugt im Bereich von 1 bis 600 µm, besonders bevorzugt im Bereich von 2 bis 400 µm, ganz besonders bevorzugt im Bereich von 5 bis 200 µm, insbesondere im Bereich von 10 bis 100 µm, abzustrahlen.

Zudem kann die Vorrichtung, insbesondere der Laser der Vorrichtung, dazu konfiguriert sein, die elektromagnetische Strahlung gleichzeitig oder sequentiell, auf mindestens eine weitere biologische Zelle, optional mehrere weitere biologische Zellen, abzustrahlen.

Der Detektor zur Detektion einer räumlichen Streckung kann ausgewählt sein aus der Gruppe bestehend aus Mikroskop, Kamera, und Kombinationen hiervon, wobei der Detektor bevorzugt eine Hochgeschwindigkeits-Kamera ist, wobei unter einer Hochgeschwindigkeitskamera eine Kamera verstanden wird, die 1 bis 10 000 Bilder pro Sekunde aufnimmt, bevorzugt 2 bis 2000 Bilder pro Sekunde, besonders bevorzugt 10 bis 1000 Bilder pro Sekunde, insbesondere 20 bis 500 Bilder pro Sekunde.

Die mindestens eine biologische Zelle kann ausgewählt sein aus der Gruppe bestehend aus Prokaryotenzelle, Archaeenzelle und Eukaryotenzelle, wobei die mindestens eine biologische Zelle bevorzugt eine Eukaryotenzelle ist, optional eine kranke Eukaryotenzelle, insbesondere eine Eukaryotenzelle, die ausgewählt ist aus der Gruppe bestehend aus Epithelzelle, Lymphozyt, Makrophage, Fibroblast, PC12-Zelle, Keratinocyt und Melanomzelle.

In einer bevorzugten Ausgestaltungsform der erfindungsgemäßen Vorrichtung liegt die optische Achse des Lasers und die optische Achse des Detektors in einem einzelnen optischen Strahlengang. Die Vorrichtung benötigt somit in einer Richtung senkrecht zu den beiden optischen Achsen wenig Raum und kann insofern sehr kompakt ausgestaltet werden.

Ferner wird erfindungsgemäß ein Verfahren zur Detektion einer räumlichen Streckung von mindestens einer adhärenten Zelle bereitgestellt, umfassend die Schritte
a) Bestrahlen von mindestens einer biologischen Zelle auf einem Substrat mit elektromagnetischer Strahlung in einer Bestrahlungsrichtung mit einem Laser, wobei die elektromagnetische Strahlung des Lasers so gewählt wird, dass sie eine räumliche Streckung der mindestens einen biologischen Zelle in eine Richtung parallel zur Bestrahlungsrichtung bewirkt;
b) Detektion einer räumlichen Streckung der mindestens einen biologischen Zelle in einer Richtung parallel zur Bestrahlungsrichtung über einen Detektor;
wobei die mindestens eine biologische Zelle eine auf dem Substrat adhärente biologische Zelle ist, dadurch gekennzeichnet, dass der Laser elektromagnetische Strahlung mit einer Leistung im Bereich von 50 bis 2000 mW abstrahlt, wobei in dem Verfahren kein Laser eingesetzt wird, der elektromagnetische Strahlung auf die mindestens eine biologische Zelle in einer Richtung strahlt, die einen Winkel von 180° zur Bestrahlungsrichtung aufweist.

Durch das erfindungsgemäße Verfahren ist es erstmalig möglich, einen Teil von adhärenten biologischen Zellen, eine ganze adhärente biologische Zelle oder mehrere adhärente biologische Zellen räumlich und zeitlich selektiv und ohne Kontaktierung mit einem Messgerät über eine gezielt applizierte Streckungskraft in eine Richtung senkrecht zum Substrat und vom Substrat weg zu Verformen, den Grad der Verformung zu detektieren und aus dem Grad der Verformung mechanische Eigenschaften (z.B. viskoelastischen Eigenschaften über Spannungs-Dehnungskurven) der mindestens einer adhärenten Zelle, oder Teilen davon, in dem natürlichen (d.h. adhärenten) Zustand der Zelle abzuleiten.

Weist die vom Substrat abgewandte Umgebung der mindestens einen biologischen Zelle einen Brechungsindex auf, der kleiner ist als der Brechungsindex der Zelle selbst (z.B. Luft mit Brechungsindex 1,00 oder wässriger Puffer mit Brechungsindex 1,33), wird die mindestens eine Zelle vom Substrat weg gestreckt. Weist die vom Substrat abgewandte Umgebung der mindestens einen biologischen Zelle einen Brechungsindex auf, der größer ist als der Brechungsindex der Zelle selbst (z.B. Glycerin mit Brechungsindex 1,47), wird die mindestens eine Zelle zum Substrat hin gestreckt, d.h. zum Substrat hin gestaucht. In beiden Fällen lassen sich durch den detektierten Grad der Verformung der mindestens einen adhärenten Zelle mechanische Eigenschaften der mindestens einen adhärenten Zelle, oder Teilen davon, ableiten.

Das erfindungsgemäße Verfahren kann dadurch gekennzeichnet sein, dass kein Laser eingesetzt wird, der elektromagnetische Strahlung auf die mindestens eine biologische Zelle in einer Richtung strahlt, die einen Winkel im Bereich von > 90° bis 180° zur Bestrahlungsrichtung aufweist.

In einer bevorzugten Ausführungsform wird in dem Verfahren eine Lichtquelle eingesetzt und die mindestens eine biologischen Zelle mit Licht in einer Lichtbestrahlungsrichtung bestrahlt, wobei die Lichtbestrahlungsrichtung bevorzugt parallel zur Bestrahlungsrichtung des Lasers auf die mindestens eine biologische Zelle ist und/oder der Bestrahlungsrichtung des Lasers auf die mindestens eine biologische Zelle entgegen gerichtet ist (d.h. die Lichtquelle in einem Winkel von 180° zur Bestrahlungsrichtung des Lasers auf die mindestens eine biologische Zelle gestrahlt wird). Die Lichtquelle ist bevorzugt ausgewählt aus der Gruppe bestehend aus Lichtquellen von inkohärentem Licht, besonders bevorzugt ausgewählt aus der Gruppe bestehend aus Halogenlampe, LED und Kombinationen hiervon.

In einer bevorzugten Ausführungsform des Verfahrens wird die elektromagnetische Strahlung, optional abgelenkt über einen dichroitischen und/oder einen einfachen Spiegel, senkrecht zu einer Oberfläche des Substrats, auf der sich die mindestens eine biologische Zelle befindet, auf die mindestens eine biologische Zelle (bzw. Teile davon) gestrahlt. Der dichroitische Spiegel kann dazu geeignet sein, Licht mit einer Wellenlänge > 700 nm (z.B. Laserlicht der Wellenlänge 800 nm) zu reflektieren und Licht mit einer Wellenlänge von ≤ 700 nm (z.B. sichtbares Licht) passieren zu lassen. Der einfache Spiegel kann dazu geeignet sein, sichtbares Licht und IR-Licht (z.B. Licht mit einer Wellenlänge von 400 nm bis 800 nm) zu reflektieren.

In einer besonders bevorzugten Ausführungsform des Verfahrens wird die elektromagnetische Strahlung durch das Substrat auf die mindestens eine biologische Zelle (oder Teile davon) gestrahlt. In diesem Fall ist es essentiell, dass das Substrat die elektromagnetische Strahlung des Lasers zumindest teilweise passieren lässt, wobei das Substrat besonders bevorzugt transparent für die elektromagnetische Strahlung ist. Das Substrat kann ausgewählt sein aus der Gruppe bestehend aus Glas, Quarzglas, Quarz, transparenter Kunststoff (z.B. PMMA oder Polycarbonat) und Kombinationen hiervon.

In einer bevorzugten Ausführungsform wird der Laser entlang einer Oberfläche des Substrats bewegt und/oder das Substrat entlang einer Oberfläche des Substrats bewegt, bevorzugt entlang einer Ebene, die parallel zu einer Oberfläche des Substrats ist. Hierfür kann ein Piezo-Tisch eingesetzt werden, auf dem das Substrat angeordnet wird.

In dem Verfahren kann eine Recheneinheit eingesetzt werden, die Daten des Detektors und/oder eines Datenspeichers der Vorrichtung empfängt und auswertet, wobei die Recheneinheit bevorzugt eine Umwandlung der Daten zu einer räumlichen Streckung der mindestens einen Zelle in unbekannter Länge in eine räumliche Streckung der mindestens einen Zelle in bekannter Länge durchführt, wobei die Recheneinheit besonders bevorzugt für die Umwandlung am Detektor detektierte Beugungsbilder und eine (kalibrierte) Punktspreizfunktion nutzt und die Recheneinheit ganz besonders bevorzugt die Daten zur räumlichen Streckung in eine fokale Höhe, ein E-Modul und/oder Schubmodul der mindestens einen biologischen Zelle umwandelt.

Für eine Erfassung der relativen Höhenänderung der mindestens einen Zelle mit hoher Sensitivität ist es vorteilhaft, wenn die Zellmembran der mindestens einen Zelle mit einer Substanz markiert ist oder wird, die einen größeren Brechungsindex aufweist als die von dem Substrat abgewandten Umgebung der mindestens einen Zelle, bevorzugt einen Brechungsindex, der größer ist als der Brechungsindex der mindestens einen Zelle. Beispielsweise können hierfür an die Zellmembran der Zelle(n) Partikel adhäriert sein oder werden. Die Partikel können beispielsweise Kunststoff, bevorzugt Polystyrol (Brechungsindex ca. 1,58), enthalten oder daraus bestehen. Bevorzugt haben die Partikel einen mittleren Partikeldurchmesser im Bereich von 1 nm bis 10 µm, besonders bevorzugt 10 nm bis 5 µm, ganz besonders bevorzugt 100 nm bis 2 µm, insbesondere 1 µm, gemessen über dynamische Lichtstreuung. Insbesondere ist die mindestens eine Zelle mit 1 bis 10, bevorzugt 2 bis 9, besonders bevorzugt 4 bis 8, Partikeln markiert. Die Detektionssensitivität der Streckung der mindestens einen Zelle lässt sich somit erheblich steigern, da die Bewegung der Partikel über den Detektor erfasst werden kann und nicht die Bewegung der (unmarkierten) Zellmembran über den Detektor erfasst werden muss.

Zur Umwandlung der vom Detektor erfassten relativen Höhenänderung in eine absolute Höhenänderung ist der Detektor in einer bevorzugten Ausgestaltungsform des Verfahrens kalibrierbar. In dieser Hinsicht wird das Substrat bevorzugt in einer Richtung parallel zur Bestrahlungsrichtung des Lasers bewegt, besonders bevorzugt über einen Piezo-Tisch, auf dem das Substrat angeordnet wird oder ist. Durch die Bewegung des Substrats in eine Richtung parallel zur Bestrahlungsrichtung des Lasers kann jedem Detektorsignal ein bestimmter Wert der Höhenänderung zugeordnet werden und somit die an sich nur qualitative Höhenänderung der Zelle(n) quantifiziert werden (z.B. in Nanometer bis Mikrometer angegeben werden).

In dem Verfahren kann eine Temperiereinheit zur Temperierung des Substrats eingesetzt werden. Bevorzugt temperiert die Temperiereinheit das Substrat auf eine Temperatur im Bereich von >0°C bis <60°C, bevorzugt 5°C bis 50 °C, besonders bevorzugt 10°C bis 45°C, insbesondere 20°C bis 40°C. Das in dem Verfahren eingesetzte Substrat kann ein Kanalsystem enthalten, das von einer Kühlflüssigkeit der Temperiereinheit durchströmt wird. Das in dem Verfahren eingesetzte Substrat kann eine spezifische Wärmekapazität von weniger als 4 J/g·K, bevorzugt weniger als 3 J/g·K, besonders bevorzugt weniger als 2 J/g·K, ganz besonders bevorzugt weniger als 1 J/g·K, insbesondere weniger als 0,5 J/g·K, aufweisen. Je niedriger die spezifische Wärmekapazität des Substrats ist, desto schneller lässt sich die Temperatur des Substrats und damit der auf dem Substrat adhärenten Zellen ändern. Es kann somit das mechanische Verhalten biologischer Zellen bei schnellen Temperatursprüngen beobachtet werden.

In einer bevorzugten Ausführungsform strahlt der Laser in dem Verfahren die elektromagnetische Strahlung mit einer Wellenlänge im Bereich von 400 bis 1200 nm, bevorzugt im Bereich von 500 bis 1100 nm, besonders bevorzugt im Bereich von 600 bis 1000 nm, insbesondere im Bereich von 700 bis 900 nm, ab.

Der Laser kann ferner in dem Verfahren die elektromagnetische Strahlung mit einer Leistung im Bereich von 200 bis 2000 mW, ganz besonders bevorzugt 1000 bis 2000 mW, abstrahlen.

Zudem kann der Laser in dem Verfahren die elektromagnetische Strahlung gepulst, bevorzugt mit einer Frequenz von 1 bis 1000 Hz, abstrahlen.

In einer weiteren bevorzugten Ausführungsform strahlt der Laser die elektromagnetische Strahlung für einen Zeitraum im Bereich von 0,05 bis 15 Sek., bevorzugt 0,2 bis 10 Sek., besonders bevorzugt 0,5 bis 5 Sek., insbesondere 1 bis 2 Sek., ab.

Das Verfahren kann sich dadurch auszeichnen, dass der Laser die elektromagnetische Strahlung in einem Winkel im Bereich von >0° bis 90°, bevorzugt im Bereich von 30° bis 90°, besonders bevorzugt im Bereich von 60° bis 90°, ganz besonders bevorzugt im Bereich von 80° bis 90°, insbesondere 90°, zu einer Oberfläche des Substrats abstrahlt.

Die vom Laser abgestrahlte elektromagnetische Strahlung kann mit einer Strahlenbreite im Bereich von 0,1 bis 800 µm, bevorzugt im Bereich von 1 bis 600 µm, besonders bevorzugt im Bereich von 2 bis 400 µm, ganz besonders bevorzugt im Bereich von 5 bis 200 µm, insbesondere im Bereich von 10 bis 100 µm, abgestrahlt werden.

Das Verfahren kann dadurch gekennzeichnet sein, dass der Laser die elektromagnetische Strahlung gleichzeitig oder sequentiell, auf mindestens eine weitere biologische Zelle, optional mehrere weitere biologische Zellen, abstrahlt.

Der im Verfahren eingesetzte Detektor zur Detektion einer räumlichen Streckung kann ausgewählt sein aus der Gruppe bestehend aus Mikroskop, Kamera, und Kombinationen hiervon, wobei der Detektor bevorzugt eine Hochgeschwindigkeits-Kamera ist, wobei unter einer Hochgeschwindigkeitskamera eine Kamera verstanden wird, die 1 bis 10 000 Bilder pro Sekunde aufnimmt, bevorzugt 2 bis 2000 Bilder pro Sekunde, besonders bevorzugt 10 bis 1000 Bilder pro Sekunde, insbesondere 20 bis 500 Bilder pro Sekunde.

Die im Verfahren eingesetzte mindestens eine biologische Zelle kann ausgewählt sein aus der Gruppe bestehend aus Prokaryotenzelle, Archaeenzelle und Eukaryotenzelle, wobei die mindestens eine biologische Zelle bevorzugt eine Eukaryotenzelle ist, optional eine kranke Eukaryotenzelle, insbesondere eine Eukaryotenzelle, die ausgewählt ist aus der Gruppe bestehend aus Epithelzelle, Lymphozyt, Makrophage, Fibroblast, PC12-Zelle, Keratinocyt und Melanomzelle.

In einer bevorzugten Ausgestaltungsform des erfindungsgemäßen Verfahrens wird die optische Achse des Lasers und die optischen Achse des Detektors in einem einzelnen optischen Strahlengang zum liegen gebracht.

Ferner wird die Verwendung der erfindungsgemäßen Vorrichtung und/oder des erfindungsgemäßen Verfahrens zur in-vitro-Diagnose einer Krankheit vorgeschlagen, wobei hierfür bevorzugt die räumliche Streckung mindestens einer zu diagnostizierenden adhärenten Zelle bestimmt wird und mit einer räumlichen Streckung von mindestens einer gesunden adhärenten Zelle und/oder mit einer räumliche Streckung von mindestens einer kranken adhärenten Zelle verglichen wird.

Anhand der nachfolgenden Figur soll der erfindungsgemäße Gegenstand näher erläutert werden, ohne diesen auf die hier dargestellte, spezifische Ausgestaltungsform einschränken zu wollen.

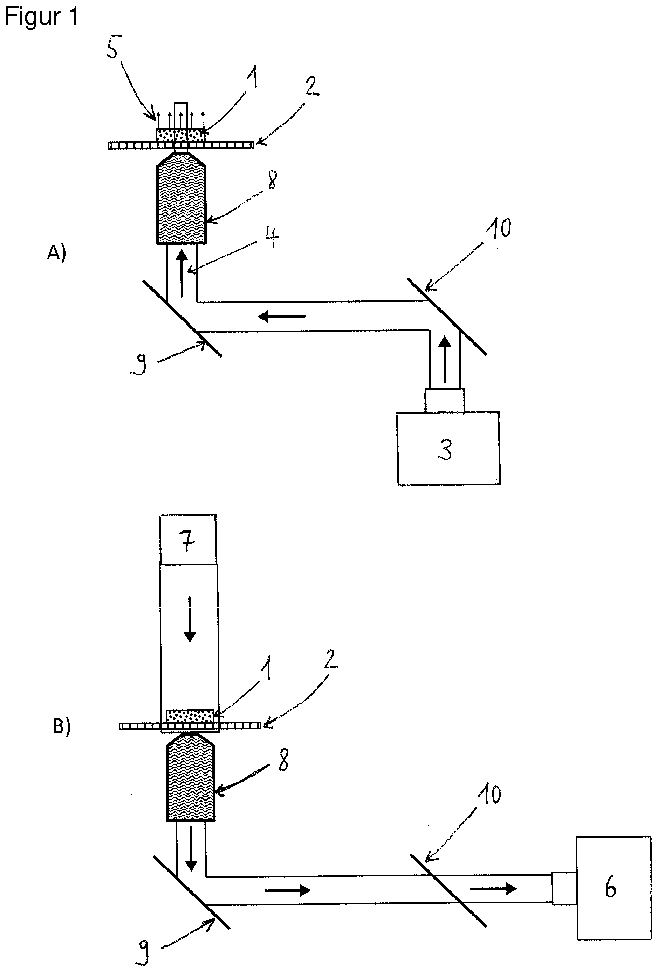

Figur 1 zeigt ein Beispiel einer erfindungsgemäßen Vorrichtung. In Figur 1A ist dargestellt, wie die räumliche Streckung der auf dem Substrat 2 adhärenten biologische Zelle 1 stattfindet. Laserlicht eines Lasers 3 wird hier zunächst über einen dichroitischen Spiegel auf einen (einfachen) Spiegel 9 abgelenkt. Der (einfache) Spiegel 9 lenkt wiederrum das Laserlicht des Lasers 3 in die Bestrahlungsrichtung 4 ab, wodurch das Laserlicht zunächst ein Objektiv 8 passiert und dann durch das (transparente) Substrat 2 auf die biologische Zelle 1 trifft. Die biologische Zelle 1 erfährt durch die Wirkung des Laserlichts eine räumliche Streckung 5. In Figur 1B ist dargestellt, wie die räumliche Streckung 5 der biologischen Zelle 1 über den Detektor 6 detektiert werden kann. In diesem Fall enthält die Vorrichtung zusätzlich eine Lichtquelle 7 zur Beleuchtung der Zelle 1, um das am Detektor 6 empfangene Lichtsignal zu verstärken. Die Zelle 1 wird durch diese Lichtquelle 7 parallel zur Bestrahlungsrichtung 4 beleuchtet. Das von der Zelle abgelenkte Licht passiert zunächst das (transparente) Substrat 2 und dann das Objektiv, um dann auf den (einfachen) Spiegel 9 zu treffen. Der (einfache) Spiegel lenkt das Licht der Zelle 1 auf den dirchroitischen Spiegel 10 ab, der das von der Lichtquelle 7 stammende Licht passieren und auf den Detektor 6 treffen lässt.

### Bezugszeichenliste

- 1:: biologische Zelle;
- 2:: Substrat;
- 3:: Laser;
- 4:: Bestrahlungsrichtung;
- 5:: räumliche Streckung der biologischen Zelle;
- 6:: Detektor zur Detektion einer räumlichen Streckung der Zelle;
- 7:: Lichtquelle zur Beleuchtung der Zelle;
- 8:: Objektiv;
- 9:: Spiegel;
- 10:: dichroitischer Spiegel.

## Patentansprüche

1. Vorrichtung zur Detektion einer räumlichen Streckung (5) von mindestens einer adhärenten Zelle (1), enthaltend
a) mindestens eine biologische Zelle (1) auf einem Substrat (2);
b) ein Laser (3) zur Bestrahlung der mindestens einen biologischen Zelle (1) mit elektromagnetischer Strahlung in einer Bestrahlungsrichtung (4), wobei der Laser (3) dazu konfiguriert ist, elektromagnetische Strahlung auf die mindestens eine biologische Zelle (1) zu strahlen, die eine räumliche Streckung (5) der mindestens einen biologischen Zelle (1) in eine Richtung parallel zur Bestrahlungsrichtung (4) bewirkt;
c) ein Detektor (6) zur Detektion einer räumlichen Streckung (5) der mindestens einen biologischen Zelle (1) in einer Richtung parallel zur Bestrahlungsrichtung (4);
wobei die mindestens eine biologische Zelle (1) eine auf dem Substrat (2) adhärente biologische Zelle (1) ist,
**dadurch gekennzeichnet, dass** der Laser (3) dazu konfiguriert ist, die elektromagnetische Strahlung mit einer Leistung im Bereich von 50 bis 2000 mW abzustrahlen,
wobei die Vorrichtung keinen Laser (3) aufweist, der dazu konfiguriert ist, elektromagnetische Strahlung auf die mindestens eine biologische Zelle (1) in einer weiteren Richtung zu strahlen, die einen Winkel von 180° zur Bestrahlungsrichtung (4) aufweist.

2. Vorrichtung gemäß dem vorhergehenden Anspruch, **dadurch gekennzeichnet, dass** die Vorrichtung keinen Laser (3) aufweist, der dazu konfiguriert ist, elektromagnetische Strahlung auf die mindestens eine biologische Zelle (1) in einer weiteren Richtung zu strahlen, die einen Winkel im Bereich von > 90° bis 180° zur Bestrahlungsrichtung (4) aufweist.

3. Vorrichtung gemäß einem der vorhergehenden Ansprüche, **dadurch gekennzeichnet, dass** die Vorrichtung dazu konfiguriert ist, den Laser (3) entlang einer Oberfläche des Substrats (2) zu bewegen und/oder das Substrat (2) entlang einer Oberfläche des Substrats (2) zu bewegen, bevorzugt entlang einer Ebene, die parallel zu einer Oberfläche des Substrats (2) ist.

4. Vorrichtung gemäß einem der vorhergehenden Ansprüche, **dadurch gekennzeichnet, dass** die Vorrichtung eine Recheneinheit aufweist, die bevorzugt dazu konfiguriert ist, Daten des Detektors (6) und/oder eines Datenspeichers der Vorrichtung empfangen und auszuwerten, wobei die Recheneinheit bevorzugt dazu konfiguriert ist, eine Umwandlung der Daten zu einer räumlichen Streckung (5) der mindestens einen Zelle (1) in unbekannter Länge in eine räumliche Streckung (5) der mindestens einen Zelle (1) in bekannter Länge umzuwandeln, wobei die Recheneinheit besonders bevorzugt dazu konfiguriert ist, für die Umwandlung am Detektor (6) detektierte Beugungsbilder und eine Punktspreizfunktion zu nutzen und die Recheneinheit ganz besonders bevorzugt dazu konfiguriert ist, die Daten zur räumlichen Streckung (5) in eine fokale Höhe, ein E-Modul und/oder Schubmodul der mindestens einen biologischen Zelle (1) umzuwandeln.

5. Vorrichtung gemäß einem der vorhergehenden Ansprüche, **dadurch gekennzeichnet, dass** die Vorrichtung eine Temperiereinheit zur Temperierung des Substrats (2) enthält, wobei bevorzugt
i) die Temperiereinheit dazu konfiguriert ist, das Substrat (2) auf eine Temperatur im Bereich von >0°C bis <60°C, bevorzugt 5°C bis 50 °C, besonders bevorzugt 10°C bis 45°C, insbesondere 20°C bis 40°C, zu temperieren; und/oder
ii) das Substrat (2) ein Kanalsystem enthält, das von einer Kühlflüssigkeit der Temperiereinheit durchströmbar ist; und/oder
iii) das Substrat (2) eine spezifische Wärmekapazität von weniger als 4 J/g·K, bevorzugt weniger als 3 J/g·K, besonders bevorzugt weniger als 2 J/g·K, ganz besonders bevorzugt weniger als 1 J/g·K, insbesondere weniger als 0,5 J/g·K, aufweist.

6. Vorrichtung gemäß einem der vorhergehenden Ansprüche, **dadurch gekennzeichnet, dass** der Laser (3) dazu konfiguriert ist, die elektromagnetische Strahlung
i) mit einer Wellenlänge im Bereich von 400 bis 1200 nm, bevorzugt im Bereich von 500 bis 1100 nm, besonders bevorzugt im Bereich von 600 bis 1000 nm, insbesondere im Bereich von 700 bis 900 nm, abzustrahlen; und/oder
ii) mit einer Leistung im Bereich von 200 bis 2000 mW, ganz besonders bevorzugt 1000 bis 2000 mW, abzustrahlen; und/oder
iii) gepulst, bevorzugt mit einer Frequenz von 1 bis 1000 Hz, abzustrahlen.

7. Vorrichtung gemäß einem der vorhergehenden Ansprüche, **dadurch gekennzeichnet, dass** der Detektor (6) zur Detektion einer räumlichen Streckung (5) ausgewählt ist aus der Gruppe bestehend aus Mikroskop, Kamera, und Kombinationen hiervon, wobei der Detektor (6) bevorzugt eine Hochgeschwindigkeits-Kamera ist, wobei unter einer Hochgeschwindigkeitskamera eine Kamera verstanden wird, die 1 bis 10 000 Bilder pro Sekunde aufnimmt, bevorzugt 2 bis 2000 Bilder pro Sekunde, besonders bevorzugt 10 bis 1000 Bilder pro Sekunde, insbesondere 20 bis 500 Bilder pro Sekunde.

8. Verfahren zur Detektion einer räumlichen Streckung (5) von mindestens einer adhärenten Zelle (1), umfassend die Schritte
a) Bestrahlen von mindestens einer biologischen Zelle (1) auf einem Substrat (2) mit elektromagnetischer Strahlung in einer Bestrahlungsrichtung (4) mit einem Laser (3), wobei die elektromagnetische Strahlung des Lasers (3) so gewählt wird, dass sie eine räumliche Streckung (5) der mindestens einen biologischen Zelle (1) in eine Richtung parallel zur Bestrahlungsrichtung (4) bewirkt;
b) Detektion einer räumlichen Streckung (5) der mindestens einen biologischen Zelle (1) in einer Richtung parallel zur Bestrahlungsrichtung (4) über einen Detektor (6);
wobei die mindestens eine biologische Zelle (1) eine auf dem Substrat (2) adhärente biologische Zelle (1) ist,
**dadurch gekennzeichnet, dass** der Laser (3) elektromagnetische Strahlung mit einer Leistung im Bereich von 50 bis 2000 mW abstrahlt,
wobei in dem Verfahren kein Laser (3) eingesetzt wird, der elektromagnetische Strahlung auf die mindestens eine biologische Zelle (1) in einer Richtung strahlt, die einen Winkel von 180° zur Bestrahlungsrichtung (4) aufweist.

9. Verfahren gemäß Anspruch 8, **dadurch gekennzeichnet, dass** in dem Verfahren kein Laser (3) eingesetzt wird, der elektromagnetische Strahlung auf die mindestens eine biologische Zelle (1) in einer Richtung strahlt, die einen Winkel im Bereich von > 90° bis 180° zur Bestrahlungsrichtung (4) aufweist.

10. Verfahren gemäß einem der Ansprüche 8 oder 9, **dadurch gekennzeichnet, dass** der Laser (3) entlang einer Oberfläche des Substrats (2) bewegt wird und/oder das Substrat (2) entlang einer Oberfläche des Substrats (2) bewegt wird, bevorzugt entlang einer Ebene, die parallel zu einer Oberfläche des Substrats (2) ist.

11. Verfahren gemäß einem der Ansprüche 8 bis 10, **dadurch gekennzeichnet, dass** in dem Verfahren eine Recheneinheit eingesetzt wird, die Daten des Detektors (6) und/oder eines Datenspeichers der Vorrichtung empfängt und auswertet, wobei die Recheneinheit bevorzugt eine Umwandlung der Daten zu einer räumlichen Streckung (5) der mindestens einen Zelle (1) in unbekannter Länge in eine räumliche Streckung (5) der mindestens einen Zelle (1) in bekannter Länge durchführt, wobei die Recheneinheit besonders bevorzugt für die Umwandlung am Detektor (6) detektierte Beugungsbilder und eine Punktspreizfunktion nutzt und die Recheneinheit ganz besonders bevorzugt die Daten zur räumlichen Streckung (5) in eine fokale Höhe, ein E-Modul und/oder Schubmodul der mindestens einen biologischen Zelle (1) umwandelt.

12. Verfahren gemäß einem der Ansprüche 8 bis 11, **dadurch gekennzeichnet, dass** in dem Verfahren eine Temperiereinheit zur Temperierung des Substrats (2) eingesetzt wird, wobei bevorzugt
i) die Temperiereinheit das Substrat (2) auf eine Temperatur im Bereich von >0°C bis <60°C, bevorzugt 5°C bis 50 °C, besonders bevorzugt 10°C bis 45°C, insbesondere 20°C bis 40°C, temperiert; und/oder
ii) das Substrat (2) ein Kanalsystem enthält, das von einer Kühlflüssigkeit der Temperiereinheit durchströmt wird; und/oder
iii) das Substrat (2) eine spezifische Wärmekapazität von weniger als 4 J/g·K, bevorzugt weniger als 3 J/g·K, besonders bevorzugt weniger als 2 J/g·K, ganz besonders bevorzugt weniger als 1 J/g·K, insbesondere weniger als 0,5 J/g·K, aufweist.

13. Verfahren gemäß einem der Ansprüche 8 bis 12, **dadurch gekennzeichnet, dass** der Laser (3) elektromagnetische Strahlung
i) mit einer Wellenlänge im Bereich von 400 bis 1200 nm, bevorzugt im Bereich von 500 bis 1100 nm, besonders bevorzugt im Bereich von 600 bis 1000 nm, insbesondere im Bereich von 700 bis 900 nm, abstrahlt; und/oder
ii) mit einer Leistung im Bereich von 200 bis 2000 mW, ganz besonders bevorzugt 1000 bis 2000 mW, abstrahlt; und/oder
iii) gepulst, bevorzugt mit einer Frequenz von 1 bis 1000 Hz, abstrahlt.

14. Verfahren gemäß einem der Ansprüche 8 bis 13, **dadurch gekennzeichnet, dass** der Detektor (6) zur Detektion einer räumlichen Streckung (5) ausgewählt ist aus der Gruppe bestehend aus Mikroskop, Kamera, und Kombinationen hiervon, wobei der Detektor (6) bevorzugt eine Hochgeschwindigkeits-Kamera ist, wobei unter einer Hochgeschwindigkeitskamera eine Kamera verstanden wird, die 1 bis 10 000 Bilder pro Sekunde aufnimmt, bevorzugt 2 bis 2000 Bilder pro Sekunde, besonders bevorzugt 10 bis 1000 Bilder pro Sekunde, insbesondere 20 bis 500 Bilder pro Sekunde.

15. Verwendung der Vorrichtung gemäß einem der Ansprüche 1 bis 7 und/oder des Verfahrens gemäß einem der Ansprüche 8 bis 14 zur *in-*vitro-Diagnose einer Krankheit, wobei hierfür bevorzugt die räumliche Streckung (5) mindestens einer zu diagnostizierenden adhärenten Zelle (1) bestimmt wird und mit einer räumlichen Streckung (5) von mindestens einer gesunden adhärenten Zelle (1) und/oder mit einer räumliche Streckung (5) von mindestens einer kranken adhärenten Zelle (1) verglichen wird.

## Claims

1. Device for detecting a spatial extension (5) of at least one adherent cell (1), comprising
a) at least one biological cell (1) on a substrate (2);
b) a laser (3) for irradiating the at least one biological cell (1) with electromagnetic radiation in an irradiation direction (4), wherein the laser (3) is configured to irradiate electromagnetic radiation onto the at least one biological cell (1), which causes a spatial elongation (5) of the at least one biological cell (1) in a direction parallel to the irradiation direction (4);
c) a detector (6) for detecting a spatial elongation (5) of the at least one biological cell (1) in a direction parallel to the irradiation direction (4);
wherein the at least one biological cell (1) is a biological cell (1) adhering to the substrate (2),
**characterised in that** the laser (3) is configured to emit the electromagnetic radiation with a power in the range from 50 to 2000 mW,
wherein the device does not have a laser (3) configured to emit electromagnetic radiation onto at least one biological cell (1) in a further direction which is at an angle of 180° to the irradiation direction (4).

2. Device according to the preceding claim, **characterised in that** the device does not have a laser (3) configured to emit electromagnetic radiation onto the at least one biological cell in a further direction which has an angle of > 90° to 180° to the irradiation direction (4).

3. Device according to one of the preceding claims, **characterised in that** the device is configured to move the laser (3) along a surface of the substrate (2) and/or to move the substrate (2) along a surface of the substrate (2), preferably along a plane that is parallel to a surface of the substrate (2).

4. Device according to one of the preceding claims, **characterised in that** the device has a computing unit which is preferably configured to receive and evaluate data from the detector (6) and/or a data memory of the device, wherein the computing unit is preferably configured to convert the data into a spatial extension (5) of the at least one cell (1) of unknown length into a spatial extent (5) of the at least one cell of known length, wherein the computing unit is particularly preferably configured to use diffraction images detected at the detector (6) and a point spread function for the conversion, and the computing unit being particularly preferably configured to convert the data for the spatial extension (5) into a focal height, an E-modulus and/or shear modulus of the at least one biological cell (1).

5. Device according to one of the preceding claims, **characterised in that** the device includes a temperature control unit for controlling the temperature of the substrate (2), wherein, preferably
i) the temperature control unit is configured to control the temperature of the substrate (2) to a temperature in the range from >0°C to <60°C, preferably 5°C to 50°C, particularly preferably 10°C to 45°C, especially 20°C to 40°C; and/or
ii) the substrate (2) contains a channel system through which a cooling liquid from the temperature control unit can flow; and/or
iii) the substrate (2) has a specific heat capacity of less than 4 J/g·K, preferably less than 3 J/g·K, particularly preferably less than 2 J/g·K, very particularly preferably less than 1 J/g·K, in particular less than 0.5 J/g·K.

6. Device according to one of the preceding claims, **characterised in that** the device, in particular the laser (3), is configured to emit electromagnetic radiation
i) with a wavelength in the range from 400 to 1200 nm, preferably in the range from 500 to 1100 nm, particularly preferably in the range from 600 to 1000 nm, especially in the range from 700 to 900 nm; and/or
ii) with a power in the range of 200 to 2000 mW, particularly preferred 1000 to 2000 mW; and/or
iii) pulsed, preferably with a frequency of 1 to 1000 Hz.

7. Device according to one of the preceding claims, **characterised in that** the detector (6) for detecting a spatial extension (5) is selected from the group consisting of a microscope, a camera, and combinations thereof, wherein the detector (6) is preferably a high-speed camera, wherein a high-speed camera is understood to be a camera that captures 1 to 10,000 images per second, preferably 2 to 2,000 images per second, particularly preferably 10 to 1,000 images per second, especially 20 to 500 images per second.

8. Method for detecting a spatial elongation (5) of at least one adherent cell (1), comprising the steps of
a) irradiating at least one biological cell (1) on a substrate (2) with electromagnetic radiation in an irradiation direction (4) using a laser (3), wherein the electromagnetic radiation of the laser (3) is selected such that it causes a spatial elongation (5) of the at least one biological cell (1) in a direction parallel to the irradiation direction (4);
b) detecting a spatial elongation (5) of the at least one biological cell (1) in a direction parallel to the irradiation direction (4) via a detector (6);
wherein the at least one biological cell (1) is a biological cell (1) adhering to the substrate,
**characterised in that** the laser (3) emits electromagnetic radiation with a power in the range from 50 to 2000 mW,
wherein no laser (3) is used in the method which emits electromagnetic radiation onto the at least one biological cell (1) in a direction which is at an angle of 180° to the irradiation direction (4).

9. Method according to claim 8, **characterised in that** the method does not use a laser (3) that emits electromagnetic radiation onto the at least one biological cell (1) in a direction that forms an angle in the range from > 90° to 180° to the irradiation direction (4).

10. Method according to one of claims 8 or 9, **characterised in that** the laser (3) is moved along a surface of the substrate (2) and/or the substrate (2) is moved along a surface of the substrate (2), preferably along a plane that is parallel to a surface of the substrate (2).

11. Method according to one of claims 8 to 10, **characterised in that** a computing unit is used in the method, which receives and evaluates data from the detector (6) and/or a data memory of the device, wherein the computing unit preferably converts the data into a spatial extension (5) of the at least one cell (1) of unknown length into a spatial extension (5) of the at least one cell (1) of known length, wherein the computing unit particularly prefers to use diffraction images detected at the detector (6) and a point spread function for the conversion, and the computing unit particularly prefers to convert the data on spatial extension (5) into a focal height, an E-modulus and/or shear modulus of the at least one biological cell (1).

12. Method according to one of claims 8 to 11, **characterised in that** a temperature control unit is used in the method to control the temperature of the substrate (2), wherein preferably
i) the temperature control unit controls the temperature of the substrate (2) to a temperature in the range from >0°C to <60°C, preferably 5°C to 50°C, particularly preferably 10°C to 45°C, especially 20°C to 40°C; and/or
ii) the substrate (2) contains a channel system through which a cooling liquid from the temperature control unit flows; and/or
iii) the substrate (2) has a specific heat capacity of less than 4 J/g·K, preferably less than 3 J/g·K, particularly preferably less than 2 J/g·K, very particularly preferably less than 1 J/g·K, in particular less than 0.5 J/g·K.

13. Method according to one of claims 8 to 12, **characterised in that** the laser (3) emits electromagnetic radiation
i) with a wavelength in the range from 400 to 1200 nm, preferably in the range from 500 to 1100 nm, particularly preferably in the range from 600 to 1000 nm, especially in the range from 700 to 900 nm; and/or
ii) with a power in the range from 200 to 2000 mW, most preferably 1000 to 2000 mW; and/or
iii) emits pulsed radiation, preferably with a frequency of 1 to 1000 Hz.

14. Method according to one of claims 8 to 13, **characterised in that** the detector (6) for detecting a spatial extension (5) is selected from the group consisting of a microscope, camera, and combinations thereof, wherein the detector is preferably a high-speed camera, wherein a high-speed camera is understood to be a camera that captures 1 to 10,000 images per second, preferably 2 to 2000 images per second, particularly preferably 10 to 1000 images per second, especially 20 to 500 images per second.

15. Use of the device according to one of claims 1 to 7 and/or the method according to one of claims 8 to 14 for the in vitro diagnosis of a disease, wherein for this purpose the spatial extension (5) of at least one adherent cell (1) to be diagnosed is determined and compared with a spatial extension (5) of at least one healthy adherent cell (1) and/or with a spatial extension (5) of at least one diseased adherent cell.

## Revendications

1. Dispositif pour détecter l'étirement spatial (5) d'au moins une cellule adhérente (1), comprenant
a) au moins une cellule biologique (1) sur un substrat (2);
b) un laser (3) pour irradier la au moins une cellule biologique (1) avec un rayonnement électromagnétique dans une direction d'irradiation (4), le laser (3) étant configuré pour émettre un rayonnement électromagnétique sur la au moins une cellule biologique (1), qui provoque un étirement spatial (5) de la au moins une cellule biologique (1) dans une direction parallèle à la direction d'irradiation (4);
c) un détecteur (6) pour détecter un étirement spatial (5) de la au moins une cellule biologique (1) dans une direction parallèle à la direction d'irradiation (4);
la au moins une cellule biologique (1) étant une cellule biologique (1) adhérant au substrat (2),
**caractérisé en ce que** le laser (3) est configuré pour émettre le rayonnement électromagnétique avec une puissance comprise entre 50 et 2000 mW,
le dispositif ne comportant pas de laser (3) étant configuré pour émettre un rayonnement électromagnétique sur au moins une cellule biologique (1) dans une autre direction qui forme un angle de 180° par rapport à la direction d'irradiation (4).

2. Dispositif selon la revendication précédente, **caractérisé en ce que** le dispositif ne comporte pas de laser (3) configuré pour émettre un rayonnement électromagnétique sur la au moins une cellule biologique dans une autre direction qui forme un angle de > 90° à 180° par rapport à la direction d'irradiation (4).

3. Dispositif selon l'une des revendications précédentes, **caractérisé en ce que** le dispositif est configuré pour déplacer le laser (3) le long d'une surface du substrat (2) et/ou pour déplacer le substrat (2) le long d'une surface du substrat (2), de préférence le long d'un plan parallèle à une surface du substrat (2).

4. Dispositif selon l'une des revendications précédentes, **caractérisé en ce que** le dispositif comprend une unité de calcul qui est de préférence configurée pour recevoir et évaluer des données du détecteur (6) et/ou d'une mémoire de données du dispositif, l'unité de calcul étant de préférence configurée pour convertir les données en une extension spatiale (5) de la au moins une cellule (1) de longueur inconnue en une extension spatiale (5) de la au moins une cellule de longueur connue, l'unité de calcul étant particulièrement configurée pour utiliser, pour la conversion, des images de diffraction détectées au niveau du détecteur (6) et une fonction de dispersion ponctuelle pour la conversion, et l'unité de calcul étant de manière tout à fait particulièrement configurée pour convertir les données relatives à l'étirement spatial en une hauteur focale, un module d'Young et/ou un module de Shear de la au moins une cellule biologique (1).

5. Dispositif selon l'une des revendications précédentes, **caractérisé en ce que** le dispositif comprend une unité de thermorégulation pour réguler la température du substrat (2), de préférence
i) l'unité de thermorégulation est configurée pour réguler la température du substrat (2) comprise entre >0 °C et <60 °C, de préférence entre 5 °C et 50 °C, de manière particulièrement préférée entre 10 °C et 45 °C, et en particulier entre 20°C et 40°C ; et/ou
ii) le substrat (2) comprend un système de canaux dans lesquels peut circuler un liquide de refroidissement provenant de l'unité de régulation de température; et/ou
iii) le substrat (2) présente une capacité thermique spécifique inférieure à 4 J/g·K, de préférence inférieure à 3 J/g·K, de manière particulièrement préférée inférieure à 2 J/g·K, de manière tout à fait particulièrement préférée inférieure à 1 J/g·K, en particulier inférieure à 0,5 J/g·K.

6. Dispositif selon l'une des revendications précédentes, **caractérisé en ce que** le dispositif, en particulier le laser (3), est configuré pour émettre le rayonnement électromagnétique
i) avec une longueur d'onde comprise entre 400 et 1200 nm, de préférence entre 500 et 1100 nm, de manière particulièrement préférée entre 600 et 1000 nm, en particulier entre 700 et 900 nm; et/ou
ii) avec une puissance comprise entre 200 et 2000 mW, de préférence entre 1000 et 2000 mW; et/ou
iii) de manière pulsée, de préférence avec une fréquence comprise entre 1 et 1000 Hz.

7. Dispositif selon l'une des revendications précédentes, **caractérisé en ce que** le détecteur (6) destiné à détecter une extension spatiale (5) est choisi parmi le groupe constitué d'un microscope, d'une caméra et de combinaisons de ceux-ci, le détecteur (6) étant de préférence une caméra à grande vitesse, une caméra à grande vitesse étant une caméra qui enregistre 1 à 10 000 images par seconde, de préférence 2 à 2000 images par seconde, de manière particulièrement préférée 10 à 1000 images par seconde, en particulier 20 à 500 images par seconde.

8. Procédé de détection d'un étirement spatial (5) d'au moins une cellule adhérente, comprenant les étapes suivantes
a) irradiation d'au moins une cellule biologique sur un substrat (2) avec un rayonnement électromagnétique dans une direction d'irradiation (4) à l'aide d'un laser (3), le rayonnement électromagnétique du laser (3) étant choisi de manière à provoquer un étirement spatial (5) de la au moins une cellule biologique (1) dans une direction parallèle à la direction d'irradiation (4);
b) détection d'un étirement spatial (5) de la au moins une cellule biologique (1) dans une direction parallèle à la direction d'irradiation (4) à l'aide d'un détecteur (6);
la au moins une cellule biologique (1) étant une cellule biologique (1) adhérant au substrat,
**caractérisé en ce que** le laser (3) émet un rayonnement électromagnétique d'une puissance comprise entre 50 et 2000 mW,
aucun laser (3) n'étant utilisé dans le procédé pour émettre un rayonnement électromagnétique sur la au moins une cellule biologique (1) dans une direction formant un angle de 180° par rapport à la direction d'irradiation (4).

9. Procédé selon la revendication 8, **caractérisé en ce que** le procédé n'utilise pas de laser (3) qui émet un rayonnement électromagnétique sur la au moins une cellule biologique (1) dans une direction qui forme un angle compris entre > 90° et 180° par rapport à la direction d'irradiation (4).

10. Procédé selon l'une des revendications 8 ou 9, **caractérisé en ce que** le laser (3) est déplacé le long d'une surface du substrat (2) et/ou le substrat (2) est déplacé le long d'une surface du substrat (2), de préférence le long d'un plan parallèle à une surface du substrat (2).

11. Procédé selon l'une des revendications 8 à 10, **caractérisé en ce que** le procédé utilise une unité de calcul qui reçoit et évalue les données du détecteur (6) et/ou d'une mémoire de données du dispositif, l'unité de calcul effectuant de préférence une conversion des données en une extension spatiale (5) d'au moins une cellule (1) de longueur inconnue en une extension spatiale (5) d'au moins une cellule (1) de longueur connue, l'unité de calcul utilisant de manière particulièrement préférée pour la conversion des images de diffraction détectées au niveau du détecteur (6) et une fonction de dispersion ponctuelle, et l'unité de calcul convertissant de manière tout particulièrement préférée les données relatives à l'extension spatiale (5) en une hauteur focale, un module d'élasticité et/ou un module de cisaillement de la au moins une cellule biologique (1).

12. Procédé selon l'une des revendications 8 à 11, **caractérisé en ce que**, dans le procédé, une unité de thermorégulation est utilisée pour réguler la température du substrat (2), de préférence
i) l'unité de thermorégulation thermorégule le substrat (2) à une température comprise entre >0°C et <60°C, de préférence entre 5°C et 50°C, de manière particulièrement préférée entre 10°C et 45°C, en particulier entre 20°C et 40°C; et/ou
ii) le substrat (2) contient un système de canaux dans lequel circule un liquide de refroidissement provenant de l'unité de régulation de température; et/ou
iii) le substrat (2) présente une capacité thermique spécifique inférieure à 4 J/g·K, de préférence inférieure à 3 J/g·K, de manière particulièrement préférée inférieure à 2 J/g·K, de manière tout à fait particulièrement préférée inférieure à 1 J/g·K, en particulier inférieure à 0,5 J/g·K.

13. Procédé selon l'une des revendications 8 à 12, **caractérisé en ce que** le laser (3) émet un rayonnement électromagnétique
i) avec une longueur d'onde comprise entre 400 et 1200 nm, de préférence entre 500 et 1100 nm, de manière particulièrement préférée entre 600 et 1000 nm, en particulier entre 700 et 900 nm; et/ou
ii) avec une puissance comprise entre 200 et 2000 mW, de préférence entre 1000 et 2000 mW; et/ou
iii) émet des impulsions, de préférence avec une fréquence comprise entre 1 et 1000 Hz.

14. Procédé selon l'une des revendications 8 à 13, **caractérisé en ce que** le détecteur (6) destiné à détecter un étirement spatial (5) est choisi parmi le groupe constitué d'un microscope, d'une caméra, et des combinaisons de ceux-ci, le détecteur étant de préférence une caméra à grande vitesse, une caméra à grande vitesse étant une caméra qui enregistre 1 à 10 000 images par seconde, de préférence 2 à 2000 images par seconde, de manière particulièrement préférée 10 à 1000 images par seconde, en particulier 20 à 500 images par seconde.

15. Utilisation du dispositif selon l'une des revendications 1 à 7 et/ou du procédé selon l'une des revendications 8 à 14 pour le diagnostic in vitro d'une maladie, pour lequel on détermine de préférence l'extension spatiale (5) d'au moins une cellule adhérente (1) à diagnostiquer et comparée à l'extension spatiale (5) d'au moins une cellule adhérente saine (1) et/ou à l'extension spatiale (5) d'au moins une cellule adhérente malade.
